Europäisches Patentamt

European Patent Office

Office européen des brevets

(19)

(11) Numéro de publication: **0 008 975**
**B1**

(12)

# FASCICULE DE BREVET EUROPEEN

(45) Date de publication du fascicule de brevet: **16.09.81**

(51) Int. Cl.³: **C 22 C 5/02, A 61 K 6/04**

(21) Numéro de dépôt: **79400578.5**

(22) Date de dépôt: **20.08.79**

(54) **Alliage jaune à base d'or pouvant être utilisé notamment comme alliage dentaire revêtu éventuellement d'un matériau céramique et procédé de durcissement d'un tel alliage.**

(30) Priorité: **31.08.78 FR 7825226**

(43) Date de publication de la demande:
**19.03.80 Bulletin 80/6**

(45) Mention de la délivrance du brevet:
**16.09.81 Bulletin 81/37**

(84) Etats Contractants Désignés:
**BE DE FR GB IT NL**

(56) Documents cités:
**CH - A - 488 017**

(73) Titulaire: **COMPTOIR LYON-ALEMAND - LOUYOT**
**13 rue de Montmorency**
**F-75139 Paris Cédex 03 (FR)**

(72) Inventeur: **Niney, Claude**
**20 rue Mozart**
**F-91470 Limours (FR)**
Inventeur: **Guerlet, Jean-Paul**
**11 rue Paul Bert**
**F-75011 Paris (FR)**

(74) Mandataire: **Combe, André et al,**
**CABINET BEAU DE LOMENIE 55 rue d'Amsterdam**
**F-75008 Paris (FR)**

Courier Press, Leamington Spa, England.

# 0 008 975

Alliage jaune à base d'or pouvant être utilisé notamment comme alliage dentaire revêtu éventuellement d'un matériau céramique et procédé de durcissement d'un tel alliage

La présente invention concerne un alliage jaune à base d'or pouvant être utilise notamment comme alliage dentaire, revêtu éventuellement d'un matériau céramique.

On recherche des alliages dentaires précieux à couler pour la technique céramométallique, présentant un ensemble déterminé de propriétés physicochimiques dont certaines sont plus ou moins compatibles.

Parmi les propriétés chimiques, on peut citer: l'absence de toxicité, la résistance à la corrosion, la qualité de la liaison céramométallique.

Parmi les propriétés physiques, on peut citer: l'intervalle de fusion, le coefficient d'expansion thermique, la conductibilité thermique.

L'alliage doit présenter en outre des propriétés mécaniques convenables caractérisées par le module d'élasticité, les limites élastiques et de rupture, l'allongement et surtout la dureté. Il faut en outre que l'alliage présente des propriétés convenables de fusion, de coulabilité, de précision des coulées, d'usinabilité, de compatibilité avec les revêtements céramiques dentaires et de couleur.

Une des caractéristiques très importantes des alliages dentaires est en effet la couleur.

Pour la réalisation d'une prothèse dentaire nécessitant de très bonnes propriétés mécaniques, telle que la réalisation d'un bridge de longue portée, on utilisait le plus souvent des alliages gris plus résistants mécaniquement que les alliages jaunes connus. Ces alliages gris présentent toutefois des inconvénients sérieux sur le plan de l'esthétique.

En effet, il est souvent difficile, compte tenu de la couleur grise du substrat, d'obtenir les nuances de couleur désirées de céramique; il est alors nécessaire d'augmenter l'épaisseur de la couche de porcelaine avec les inconvénients techniques que en résultent, compte tenu des impératifs particuliers d'épaisseur et d'alignement.

En outre, si on veut laisser une partie visible du bridge non revêtu de porcelaine, il est alors nécessaire de juxtaposer et de braser un élément en un alliage jaune plus esthétique.

Des alliages dentaires d'or tendant à regrouper l'ensemble des propriétés indiquées ci-dessous ont été décrits dans le brevet allemand n° 1 533 233. Ces alliages comportent 5 à 15% de platine, 70 à 90% d'or, 0,5 à 10% de palladium, 0,1 à 2% d'indium, 0,1 à 2% d'étain, 0,05 à 1% de rhénium et 0,1 à 1% de fer.

Ces alliages peuvent contenir en outre jusqu'à 5% d'argent, jusqu'à 1% de cuivre, de 0,05 à 0,5% d'iridium et/ou jusqu'à 5% de zinc.

Ces alliages comportent nécessairement du rhénium. Or, le rhénium est un métal onéreux, très difficilement soluble dans l'or.

Par ailleurs, on note que les alliages spécifiquement décrits dans le brevet présentent un rapport

$$\frac{Pd}{Pt} > 0,62.$$

En outre, la somme Pt + Pd n'est jamais inférieure à 12,7%. Par conséquent, ces alliages sont blancs ou ne présentent qu'une couleur jaune très pâle, voire gris jaunâtre.

En outre, tous les alliages spécifiquement décrits comportent nécessairement 0,3% de cuivre. Or, le cuivre est connu pour colorer la couche céramique.

L'invention a pour but de proposer un alliage dentair de couleur jaune exempt de cuivre et de rhénium réalisant un compromis tel que cet alliage est particulièrement avantageux pour son application comme alliage dentaire à couler pouvant être utilisé tel quel comme prothèse ou revêtu par cuisson d'un matériau céramique adapté, du type porcelaine.

Dans tout ce qui suit, sauf indication contraire, les teneurs sont exprimées en ‰ en poids par rapport au poids total de l'alliage.

Ce but est atteint par l'invention que concerne en effet un alliage jaune à base d'or caractérisé en ce qu'il consiste en:

820 à 920‰ d'or

50 à 100‰ de platine

5 à 40‰ de palladium

2 à 20‰ d'iridium

2 à 25‰ d'indium

1 à 10‰ de fer

5 à 50‰ d'argent

et en ce que les teneurs en Pd, Pt et Ir répondent aux équations suivantes:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

$$Pd + Pt \leqslant 110\text{‰} \qquad (3)$$

On peut remplacer jusque'à 90% du poids d'iridium par un poids équivalent de rhodium. En outre, on peut remplacer jusqu'à 50% du poids d'iridium par un poids équivalent de ruthénium.

De même, on peut remplacer jusqu'à 50% du poids d'indium par de l'étain. Toutefois, l'iridium et l'indium sont les métaux preférés.

L'alliage selon l'invention peut comporter également de 1 à 10‰ d'au moins un métal choise parmi le chrome, le cobalt et le nickel. Ces métaux peuvent en fait remplacer totalement ou partiellement le fer aussi convient-il que la quantité totale de fer, chrme, cobalt et nickel dans l'alliage soit comprise entre 1 et 10‰.

Les alliages préférés ont la composition suivante:

860 à 910‰ d'or

60 à 80‰ de platine

10 à 30‰ de palladium

3 à 12‰ d'iridium

5 à 15‰ d'indium

2 à 8‰ de fer

5 à 30‰ d'argent

avec:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

L'alliage selon l'invention présente une belle couleur jaune. La couleur jaune des alliages selon l'invention est généralement très voisine de la couleur jaune des alliages d'or dentair du commerce et définis par la norme NF—S 91 204 de juin 1976. L'alliage selon l'invention est d'autant plus jaune que la teneur en platine et en palladium est plus faible.

L'alliage selon l'invention permet, grâce à sa couleur jaune, d'utiliser des couches minces de céramique et effectuer une coulée en monobloc d'un bridge dont une partie visible n'est pas revêtue de céramique et laisse ainsi apparaître l'alliage sans nuire à l'esthétique de l'ensemble, compte tenu précisément de la couleur jaune apparente de cet alliage.

Par ailleurs, l'alliage selon l'invention présente une dureté Vickers remarquable généralement supérieure à 120 HV pour les alliages bruts de coulée et supérieure à 150 HV pour les alliages ayant subi les cuissons pour le dépôt de la couche céramique.

Cette dureté s'élève généralement à plus de 180 HV, et même plus de 200 HV après un traitement isotherme supplémentaire effectué à environ 450—600°C pendant 5 à 30 min sur les alliages ayant déjà subi les traitements de cuisson de revêtement céramique.

Cette dureté remarquable est accompagnée d'une coulabilité et d'une usinabilité très satisfaisantes. Par ailleurs, les alliages selon l'invention présentent une température de fusion généralement supérieure à 1070°C, ce qui constitue un point de fusion suffisamment élevé pour le dépôt et la fixation d'une grande variété de matériaux céramiques dentaires du type porcelaine qui s'effectuent la plupart du temps à une température n'excédant pas 1010°C environ.

3

**0 008 975**

Par ailleurs, les alliages selon l'invention permettent d'obtenir une très bonne adhérence du matériau céramique dentaire, du type porcelaine, après cuisson.

La présence simultanée de fer (remplacé éventuellement en tout ou partie par au moins un élément choisi permi le chrome, le cobalt et le nickel) et d'indium (remplacé éventuellement jusqu'à 50% de son poids par un poids équivalent d'étain) est nécessaire pour obtenir un alliage présentant une dureté suffisante en une bonne liaison céramique.

Des quantités de fer et d'indium inférieures aux quantités minimales indiquées conduisent à l'obtention d'alliages trop mous, des quantités supérieures aux quantités maximales indiquées conduisent à des alliages ne présentant pas un gain sensible en dureté, mais, par contre, s'oxydant trop fortement.

Le platine durcit l'or et la quantité de palladium est choisie de façon à faciliter la mise en solution du platine dans l'or. Des quantités de palladium supérieures aux quantités maximales indiquées conduisent à un alliage trop blanc sans accroître pour cela de façon sensible la dureté de l'alliage.

L'indium et/ou l'étain et également le fer conduisent à la formation d'une couche d'oxydes permettant une bonne liaison entre l'alliage et le revêtement céramique.

Si la quantité d'iridium et de palladium est inférieure à la quantité minimale indiquée, on obtient un alliage trop mou. Par contre, une quantité d'iridium ou de rhodium supérieure à 20%$_o$ conduit à un alliage difficile à travailler.

Si la quantité d'argent ets inférieure à la quantité minimale indiquée, l'alliage n'est plus assez jaune et ses propriétés mécaniques se détériorent; de même, si la quantité d'argent est supérieure à la quantité maximale indiquée, on obtient un alliage qui ne réalise plus un bon compromis entre la couleur et les propriétés.

De ce qui précède et des exemples comparatifs ci-dessous, il apparaît que si, pour un constituant essentiel de l'alliage ou si, a fortiori, pour plus d'un constitant, on sort des compositions indiquées, on obtient un alliage qui ne présente plus le compromis avantageux de propriétés physicochimiques et de couleur désirée. On constate les mêmes faits si l'on supprime au moins un constituant.

D'autres avantages et caractéristiques de la présente invention apparaîtront à la lecture des exemples suivants donnés à titre illustratif, nullement limitatif.

Exemple 1

On fond dans un creuset en alumine et on coule un lingot ayant la composition suivante:

Au : 886%$_o$

Pt : 70%$_o$

Pd : 15%$_o$

Ir : 5%$_o$

Fe : 3%$_o$

In : 11%$_o$

Ag : 10%$_o$

Pour ce faire, on part d'un alliage mère constitué par du platine-iridium 25% auquel on rajoute les quantités adaptées de métaux purs, de façon à aboutir à la composition désirée.

Le lingot de couleur jaune est ensuite homogénéisé à 800°C pendant 1 h et on le trempe à l'eau à 20°C. On lamine le lingot de façon à obtenir un feuillard dans lequel on découpe des plots de 1 g qui servent de matière première pour la fonte et la coulée en cire perdue des prothèses dentaires.

On utilise un revêtement oxyphosphaté du commerce pour réaliser le moule à cire perdue pour couler un bridge. On porte le revêtement à 825°C pendant 40 min et on coule les plots d'alliage préalablement fondus à 1200°C dans un creuset en graphite en utilisant une fronde rotative. On laisse refroidir le métal dans le moule. La pièce est démoulée, sablée, grattée. C'est sur cette pièce, brute de coulée, que l'on effectue des mesures de dureté, dite dureté BC.

On revêt ensuite le bridge d'une céramique du commerce. Pour ce faire, on effectue les opérations suivantes:

1) oxydation du bridge à 1010°C pendant 10 min à l'air ambiant,

2) enduction d'opaque et montée en température sous vide de 650 à 930°C (50°C/min) et de 930 à 1000°C à l'air ambiant (50°C/min),

3) enduction de la céramique proprement dite et montée température de 650 à 930°C sous vide (30°C/min) et de 930 à 980°C à l'air ambiant (30°/min),

4) glaçage de l'ensemble par montée en température de 650°C à 980°C à l'air ambiant (50°C/min).

Après cuisson de la céramique, on effectue sur une partie du bridge, non revêtue de céramique,

4

une mesure de dureté de l'alliage dite ACC. Le résultat de cette mesure de dureté de l'alliage dite ACC. Le résultat de cette mesure dépendra donc des différents cycles thermiques auxquels l'alliage a été soumis lors du revêtement par la céramique, mais ne dépendra pas ou peu de la nature et de la composition de la céramique. C'est pourquoi les résultats obtenus seront sensiblement les mêmes si la mesure est effectuée sur un alliage subissant les mêmes cycles thermiques, mais sur lequel aucune céramique n'aura été déposée.

Sur une autre partie du bridge (non revêtue de céramique), on a réalisé un traitement de revenue de 30 min à 525°C; les mesures de dureté réalisées sur le produit obtenu sont dites ATR.

Les résultats obtenus sont les suivants:

| Etat de l'alliage | BC | ACC | ATR |
|---|---|---|---|
| Dureté HV (5 kg/mm²) | 130—140 | 165—185 | 185—205 |

### Exemple 1bis

On effectue les mêmes opérations que dans l'exemple 1, sauf que l'on coule l'alliage de façon à realiser trois éprouvettes conçues selon la norme DIN 13 906 d'août 1975. Une partie des éproutettes brutes de coulée obtenues subissent les mêmes cycles de cuisson que dans l'exemple 1, sans dépôt effectif de céramique et une partie de ces éprouvettes subissant en outre le traitement de revenu à 525°C pendant 30 min. On détermine sur ces éprouvettes la charge de rupture R, la limite élastique E et l'allongement A.

Les résultats obtenus sont les suivants:

| Etat des éprouvettes | BC | ACC | ATR |
|---|---|---|---|
| R (daN/mm²) | 37—41 | 44—50 | 53—60 |
| E (daN/mm²) | 26—30 | 32—38 | 44—50 |
| A (%) | 7—11 | 6—12 | 5—10 |

### Exemples 2 à 13

On effectue les mêmes opérations que dans l'exemple 1. Les compositions des alliages et les duretés obtenues sont consignées dans le tableau ci-après.

### Exemples Comparatifs 14 à 19

On effectue les mêmes opérations que dans l'exemple 1. Les compositions des alliages non conformes à l'invention et les duretés sont consignées dans le tableau ci-après.

On remarque d'après l'exemple 14 que l'absence de palladium entraîne une chute de la dureté. Il en est de même s'il n'y a pas de fer et d'indium. On note en outre, d'après les exemples 15 et 16, qu'il n'est pas souhaitable de remplacer la totalité de l'indium par l'étain. D'après les exemples 17 et 18, il apparaît que la seule absence de fer entraîne une chute de la dureté.

L'exemple 19 concerne un alliage sans argent. Cet alliage présente une dureté acceptable, mais une couleur trop pâle (pas assez jaune).

## 0 008 975

TABLEAU

| Exemples | Au | Pt | Pd | Ir | Fe | Ag | In | Divers | Dureté | | |
|---|---|---|---|---|---|---|---|---|---|---|---|
| | | | | | | | | | BC | ACC | ATC |
| 2 | 866 | 65 | 30 | 2 | 5 | 12 | 5 | Sn 5 / Rh 10 | 134 | 145 | 195 |
| 3 | 860 | 70 | 25 | 15 | 5 | 10 | 15 | | 145 | 198 | |
| 4 | 855 | 70 | 25 | 15 | 5 | 10 | 20 | | 160 | 206 | |
| 5 | 865 | 70 | 20 | 10 | 5 | 10 | 20 | | 146 | 190 | |
| 6 | 875 | 70 | 15 | 5 | 5 | 10 | 20 | | 143 | 180 | 207 |
| 7 | 877 | 70 | 15 | 5 | 3 | 10 | 20 | | 133 | 196 | 208 |
| 8 | 872 | 70 | 15 | 5 | 8 | 10 | 20 | | 162 | 186 | |
| 9 | 887 | 70 | 15 | 5 | 9 | 10 | 10 | | 135 | 170 | 191 |
| 10 | 884 | 70 | 15 | 5 | | 10 | 11 | Ni 5 | 125 | 116 | 166 |
| 11 | 884 | 70 | 15 | 5 | | 10 | 11 | Cr 5 | 129 | 113 | 176 |
| 12 | 884 | 70 | 15 | 5 | | 10 | 11 | Co 5 | 149 | 146 | 183 |
| 13 | 868 | 65 | 30 | 10 | 5 | 12 | 5 | Sn 5 | 130 | 133 | 180 |
| Exemples comparatifs | | | | | | | | | | | |
| 14 | 860 | 95 | | 15 | 5 | 12 | | Sn 13 | 93 | 105 | 139 |
| 15 | 865 | 70 | 30 | 15 | | 12 | | Sn 8 | 90 | 110 | |
| 16 | 860 | 70 | 30 | 15 | | 10 | | Sn 15 | 88 | 97 | |
| 17 | 860 | 70 | 30 | 15 | | 10 | 15 | | 133 | 93 | |
| 18 | 855 | 70 | 30 | 15 | | 10 | 20 | | 129 | 114 | |
| 19 | 894 | 70 | 15 | 5 | 5 | | 11 | | 127 | 165 | 178 |

## Revendications

1. Alliage jaune à base d'or, caractérisé en ce qu'il consiste en:

820 à 920‰ d'or

50 à 100‰ de platine

5 à 40‰ de palladium

2 à 20‰ d'iridium

2 à 25‰ d'indium

5 à 50‰ d'argent

1 à 10‰ d'au moins un métal choisi parmi le fer
le chrome, le cobalt et le nickel,

6

**0 008 975**

et en ce que les teneurs en Pd, Pt et Ir répondent aux équations suivantes:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

$$Pd + Pt \leqslant 110\text{‰} \qquad (3)$$

ledit alliage ne comportant ni cuivre, ni rhénium.

2. Alliage jaune à base d'or, caractérisé en ce qu'il consiste en:

860 à 910‰ d'or

60 à 80‰ de platine

10 à 30‰ de palladium

3 à 12‰ d'iridium

5 à 15‰ d'indium

2 à 8‰ de fer

5 à 30‰ d'argent

et en ce que:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

3. Alliage selon l'une quelconque des revendications précédentes, caractérisé en ce que jusqu'à 90% du poids d'iridium sont remplacés par un poids équivalent de rhodium.

4. Alliage selon la revendication 1 ou 2, caractérisé en ce que jusqu'à 50% du poids d'iridium sont remplacés par un poids équivalent de ruthénium.

5. Alliage selon l'une quelconque des revendications précédentes, caractérisé · en ce que jusqu'à 50% du poids d'indium sont remplacés par un poids équivalent d'étain.

6. Alliage de composition:

886‰ d'oir

70‰ de platine

15‰ de palladium

5‰ d'iridium

3‰ de fer

11‰ d'indium

10‰ d'argent.

7. Alliage dentaire caractérisé en ce qu'il a la composition d'un alliage selon l'une des revendications 1 à 6.

8. Prothèse dentaire caractérisée en ce qu'elle est constituée par un alliage selon l'une des revendications 1 à 6 recouvert d'une céramique dentaire.

9. Procédé de durcissement d'un alliage conforme à la revendications 6, caractérisé en ce que l'on effectue sur ledit alliage, après l'avoir éventuellement revêtu au moins en partie d'un revêtement

7

céramique, un traitement isotherme à une temperature comprise entre 450 et 600°C pendant 5 à 30 min.

**Claims**

1. Gold-containing yellow alloy, characterized in that it consists in:

820 to 920‰ of gold

50 to 100‰ of platinum

5 to 40‰ of palladium

2 to 20‰ of iridium

2 to 25‰ of indium

5 to 50‰ of silver

1 to 10‰ of at least a metal selected from iron, chromium, cobalt and nickel.

and in that the Pd, Pt and Ir contents correspond to the following equations:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

$$Pd + Pt \leqslant 110‰ \qquad (3)$$

the said alloy containing neither copper nor rhenium.

2. Gold-containing yellow alloy, characterized in that it consists essentially in:

860 to 910‰ of gold

60 to 80‰ of platinum

10 to 30‰ of palladium

3 to 12‰ of iridium

5 to 15‰ of indium

2 to 8‰ of iron

5 to 30‰ of silver

and in that:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

3. Alloy according to any one of the preceding claims, characterized in that up to 90% of the weight of iridium are replaced by an equivalent weight of rhodium.

4. Alloy according to claim 1 or 2, characterized in that up to 50% of the weight of iridium are replaced by an equivalent weight of ruthenium.

8

5. Alloy according to any one of the preceding claims, characterized in that up to 50% of the weight of indium are replaced by an equivalent weight of tin.

6. Alloy composed of:

886‰ of gold

70‰ of platinum

15‰ of palladium

5‰ of iridium

3‰ of iron

11‰ of indium

10‰ of silver.

7. Dental alloy characterized in that it has the composition of an alloy according to one of claims 1 to 6.

8. Dental prosthesis characterized in that it is constituted by an alloy according to one of claims 1 to 6 covered with a dental ceramic.

9. Process for hardening an alloy according to claim 6, characterized in that the said alloy, which may have been covered at least partly with a ceramic layer, is subjected to an isothermal treatment at a temperature which may vary between 450 and 600°C, for a period of 5 to 30 mins.

**Patentansprüche**

1. Gelbe Legierung auf Goldbasis, dadurch gekennzeichnet, dass sie aus:

820 bis 920‰ Gold

50 bis 100‰ Platin

5 bis 40‰ Palladium

2 bis 20‰ Iridium

2 bis 25‰ Indium

5 bis 50‰ Silber·

1 bis 10‰ von mindestens einem unter Eisen, Chrom, Kobalt und Nickel gewählten Metall

besteht und dadurch dass der Gehalt an Pd, Pt und Ir folgenden Gleichungen entspricht:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

$$Pd + Pt \leqslant 110‰ \qquad (3)$$

wobei die gennante Legierung weder Kupfer noch Rhenium enthält.

2. Gelbe Legierung auf Goldbasis, dadurch gekennzeichnet, dass sie im wesentlichen aus:

860 bis 910‰ Gold

60 bis 80‰ Platin

10 bis 30‰ Palladium

3 bis 12‰ Iridium

5 bis 15‰ Indium

2 bis 8‰ Eisen

5 bis 30‰ Silber

besteht und dadurch dass:

$$\frac{1}{10} \leqslant \frac{Pd}{Pt} \leqslant \frac{1}{3} \qquad (1)$$

$$\frac{Ir}{Pt} < \frac{1}{7} \qquad (2)$$

3. Legierung nach einem der vorhergehenden Ansprüche, dadurch gekennzeichnet, dass bis zu 90% des Gewichts an Iridium durch ein äquivalentes Gewicht an Rhodium ersetzt werden.

4. Legierung nach Anspruch 1 oder 2, dadurch gekennzeichnet, dass bis zu 50% des Gewichts an Iridium durch ein äquivalentes Gewicht an Ruthen ersetzt werden.

5. Legierung nach einem der vorhergehenden Ansprüche, dadurch gekennzeichnet, dass bis zu 50% des Gewichts an Indium durch ein äquivalentes Gewicht an Zinn ersetzt werden.

6. Legierung mit der Zusammensetzung:

886‰ Gold

70‰ Platin

15‰ Palladium

5‰ Iridium

3‰ Eisen

11‰ Indium

10‰ Silber.

7. Zahnlegierung, dadurch gekennzeichnet, dass sie die Zusammensetzung einer Legierung nach einem der Ansprüche 1 bis 6 hat.

8. Zahnprothese, dadurch gekennzeichnet, dass sie aus einer Legierung nach einem der Ansprüche 1 bis 6 besteht, die mit einer Zahnkeramik überzogen ist.

9. Härtungsverfahren für eine Legierung gemäss Anspruch 6, dadurch gekennzeichnet, dass man diese Legierung, nachdem sie eventuell mit mindenstens einem Teil eines keramischen Belags überzogen wurde, 5 bis 30 Minuten lang einer isothermischen Behandlung bei einer Temperatur zwischen 450 und 600°C unterzieht.